# EUROPEAN PATENT APPLICATION

(11) **EP 4 792 803 A1**
(43) Date of publication of application: **19.08.2026**
(21) Application number: 25158246.6
(22) Date of filing: 17.02.2025
(51) Int. Cl.: A61K 9/00, A61K 9/06, A61K 47/36, A61L 31/14

(54) **GEL-FORMING MULTI-COMPONENT COMPOSITION FOR TREATING OR PREVENTING A MEDICAL CONDITION**

(71) Applicant: Purenum GmbH, 28359 Bremen (DE)
(72) Inventor: VOLLENBRÖKER, Marc, 50670 Köln (DE); PESCHKA, Manfred, 28359 Bremen (DE)
(74) Representative: Eisenführ Speiser

(57) **Abstract**

The present invention relates to a gel-forming multi-component composition for use in a method for treating or preventing a medical condition of a subject, to such a gel-forming multi-component composition for the manufacture of a medicament, to a method for treating or preventing a medical condition of a subject, and to the use of one of the components of the composition for dissolving a medical drug.

## Description

The present invention relates to a gel-forming multi-component composition for use in a method for treating or preventing a medical condition of a subject, to such a gel-forming multi-component composition for the manufacture of a medicament, to a method for treating or preventing a medical condition of a subject, and to the use of one of the components of the composition for dissolving a medical drug.

In the field of cancer therapy, the systemic administration of chemotherapeutics is often connected with severe side-effects and low concentrations of the chemotherapeutics, which can be administered. Thus, many approaches now focus on the local administration of chemotherapeutics, for which several advantages have been reported. For example, with local administration, a higher concentration of the chemotherapeutics may be administered, since they are not (or only minimally) entering the blood circulation. Likewise, the chemotherapeutics are not diluted in the blood circulation until they reach the tumor site. Thus, the tumor can be effectively targeted with higher concentrations of chemotherapeutics. Also, since only a limited spread of the chemotherapeutics is observed, the occurrence of side-effects is usually significantly reduced in local administration.

The same applies accordingly for other medication, for example antibiotics, anti-fungal agents, anti-viral agents, which are often applied systemically and thus, the amount of medication reaching the site of infection is only a proportion of the administered dose, since the medication is distributed over the blood circulation.

Likewise, in case of pain or further conditions such as hypercholesterolemia or inflammation, specific areas of the body (e.g. the site causing the pain, the liver for hypercholesterolemia, or the site of inflammation) may be targeted by local administration. This applies to any condition, for which a particular area or site of the body can or shall be targeted.

At the same time, many conditions need to be targeted with the respective medication for long time, thus, the area or site of the body needs to be exposed with the medication for some time. For this purpose, approaches providing a sustained release of the medication have been developed. However, the approaches are usually designed for specific applications in specific areas of the body. Not all of these approaches may be applied in various areas of the body.

Particularly, areas of the body, which are in direct contact with flowing body liquids such as blood or urine, provide challenges, since the medication shall be continuously released at the same site. However, water-based options, such as solutions of the medication in water, or other known approaches are usually carried away from the target site within short time and are thus not suitable for this purpose.

For example, when treating superficial bladder cancer, usually the medication is administered intravesically. The medication should remain in the urinary bladder for 1 to 2 hours. Thus, the treatment usually begins only after micturition and requires that the patient does not drink any water or the like during the treatment, which is a major disadvantage of such a treatment option.

A treatment of the kidneys is even further complicated by the fact that the kidney function cannot be controlled by the patient (in contrast to the urinary bladder as described above). Since urine is continuously produced in the kidneys, medication will usually be washed away in short time.

As described above, the kidneys and the urinary bladder provide challenges for local and sustained exposure with medication, since they involve flowing body liquids, i.e. urine, and thus face the above disadvantages of the currently known approaches. Further, any approach in the kidneys and the urinary bladder will be in contact with urine and thus needs to be functional under these conditions, e.g. at an acidic / slightly acidic pH.

The same applies accordingly to many further areas of the body and is not limited to the kidneys and the urinary bladder. Likewise, the same challenges also arise in case an infection or inflammation shall be treated.

In the prior art, some treatment options involve providing a thermoreversible gel including the respective medication. In these options, a gel is formed at the target site, wherein a composition is applied, which has a high viscosity at lower temperatures and is a gel when reaching body temperature, or shortly before. However, the time when the composition reaches the respective temperature and is thus present as a gel, cannot be exactly controlled and depends on many factors such as the initial temperature of the composition before administration, the body temperature of the patient, or the blood circulation at the target site. Especially at sites with only low supply with blood, e.g. adipose tissue, or sites with varying supply with blood, e.g. the limbs (depending on the environmental temperature, physical activity before the treatment, etc.), the precise time of gel formation cannot be controlled. Thus, a risk of too early or too late gel formation is present, such that the site of interest is not completely covered with the gel or the composition is even carried away by blood or urine. Further, the thermoreversible gels are usually extremely soft and thus not suitable for most applications. For example, the thermoreversible gels usually cannot be gripped and thus cannot be moved for correcting their position or be removed e.g. with a gripper. Additionally, adding further composition, such as to increase or correct the applied dose, is often connected with complications.

To try to overcome this disadvantage, some options include an external device for providing heat to the composition, which may then enable the gel formation. However, these options require an additional device, which needs to be inserted into the patient's body. Further, such devices bear the risk of tissue damages either by mechanical forces or by the provided temperature.

Even further, in some cases, no gel formation occurs in the presence of urine and thus the presence of urine is even excluded from the possible applications.

Moreover, a disadvantage of thermoreversible gels is that the viscosity is affected by temperature changes. Thus, e.g. in case of reduced blood circulation, low environmental temperature, (no) physical activity, the obtained gel may liquefy due to temperature changes, which may affects the precise position of the gel and may provide uncontrolled release of the contained medication. It is thus of advantage if the formation and maintenance of a gel state is not (only) based on the temperature. However, at the same time, it is beneficial if the applied gel - once formed - does not need to be removed by another medical intervention, once the medical drug has been fully released or once the medical condition requires no further treatment with said medical drug.

Further, for some medical drugs, it is rather difficult to achieve a (complete or at least partial) dissolution in the composition, which will be applied to the patient's body. Particularly the chemotherapeutic Mitomycin C is especially difficult to dissolve and apply to the patient in such a composition.

There is thus a need for treatment options, which provide a local and sustained release of medication and which can be applied to different areas of the body.

The primary object of the present invention was thus to provide such an option, wherein the above disadvantages are possibly reduced or even avoided.

The primary object of the present invention is solved by a gel-forming multi-component composition, preferably two-component composition, preferably three-component composition, for forming a gel,
the composition comprising or consisting of
   a component (A), comprising one or more cross-linkable, preferably cationically cross-linkable polymer(s),
   wherein the or one polymer is selected from the group consisting of alginates, pectin, carrageenane, gellane, chitosan, hyaluronic acid, fibrin, gum arabicum, gellan gum, xanthan, gelatine, pullulan, fucoidane, lignin, agarose, dextran, amylose, chondroitin sulfate, keratan sulfate, heparin, lentinan, glucomannan, polygalacturonic acid, and sporopollenin,
      and
   a component (B), comprising one or more cross-linking agent(s) for cross-linking the cross-linkable polymer(s)
   preferably wherein the, one, more or all cross-linking agent(s) of component (B) is/are selected from the group consisting of mono-, bi- or tri-valent cations, preferably
   from iron, zinc, magnesium, aluminium, potassium and calcium ions, and their pharmaceutically acceptable salts, and mixtures thereof,
for use in a method for treating or preventing a medical condition of a subject,
wherein the treatment or prevention comprises administering component (A) and component (B) and optionally further component(s) to a site of interest on or in the subject's body,
wherein before the administration at least one medical drug is partially or completely dissolved in component (A) and/or component (B).

It was surprisingly found that a variety of different medical drugs can be dissolved in at least one of component (A) or (B). Particularly, the chemotherapeutic Mitomycin C, which is especially difficult to dissolve, could be dissolved, as shown in the example section, which is a major advantage for provide treatment options including Mitomycin C or other hardly soluble medical drugs.

Moreover, it was surprisingly found that the hardly soluble Mitomycin C did not negatively affect the formation of a gel with the composition according to the invention. Thus, a gel including medical drugs can be obtained with the composition according to the invention. It was particularly surprisingly found that the gel formation occurred even in synthetic urine and thus, the composition according to the invention may also be successfully applied for treatment or prevention in e.g. the kidneys or the urinary bladder.

The term "did not negatively affect the formation of a gel", as used herein, preferably describes that the formation of a gel is still possible. By adding a medical drug, as described herein, the formation of a gel may be slower, however is still considered as not negatively affected, since the formation of a gel is still observed. Particularly preferably, the formation of a gel is not significantly slower by the addition of the medical drug, preferably the time for forming a gel when a medical drug is present, as described herein, is not higher than 150%, preferably not higher than 140 %, further preferably not higher than 130 % even further preferably not higher than 120 % of the time for forming a gel under the same conditions and with the same composition, but without the medical drug.

Moreover, it was surprisingly found that the formed gel is continuously but slowly degraded in the synthetic urine. Thus, once the gel has been formed, it slowly degrades and slowly releases the included medical drug. Therefore, after the gel has been fully degraded, no residuals remain in the subject's body and no further medical intervention to remove the gel is necessary.

Depending on the administered amount of components (A) and (B), at least one of them including the medical drug as described herein, a larger or smaller gel is obtained. Larger gels require more time to be fully degraded than smaller gels. Thus, the duration of the continuous release can be controlled by the gel size and by the administered amount of components (A) and (B). Further, higher concentrations of the medical drug in the obtained gel leads to higher concentrations released per time (compared to the same gel but with lower concentrations of the medical drug). Thus, depending on the total amount of medical drug dissolved in component (A) and/or (B), the concentration of released medical drug per time can be controlled.

Consequently, with the administered amount of components (A) and (B) and the concentration of the dissolved medical drug in component (A) and/or (B), as described herein, the release rate and duration may be adjusted.

The term "gel-forming composition", as used herein, is preferably understood such that the composition is not only suitable to form a gel, but by mixing the components, a gel is actually obtained. Thus, it is preferred that when mixing of at least components (A) and (B) a gel is formed.

Preferably, the term "gel", as used herein, refers to a hydrogel. Preferably, the gel (formed by mixing the components) is a hydrogel.

Preferably, the term "multi-component composition", as used herein, describes a composition including two or more components, wherein said components are not mixed with each other, preferably are not in direct contact with each other. For example the or one, two, three or more or all components may be separated from each other, for example by at least one component being present in a container of any kind. A component may be a single substance or a mixture of any kind.

Preferably, the gel-forming multi-component composition according to the invention comprises the at least one medical drug, as described herein, as one component, e.g. component (D) and/or preferably component (A) and/or (B) comprises the at least one medical drug.

Preferably, the or one polymer in component (A) is or comprises an alginate, preferably sodium alginate, preferably wherein the cross-linking agent in component (B) is or comprises Ca²⁺.

Preferably, the or one polymer in component (A) is or comprises pectin, preferably wherein the cross-linking agent in component (B) is or comprises Ca²⁺.

Preferably, the or one polymer in component (A) is or comprises carrageenane, preferably wherein the cross-linking agent in component (B) is or comprises K⁺, preferably wherein the cross-linking agent in component (B) is or comprises Ca²⁺.

Preferably, the or one polymer in component (A) is or comprises gellane, preferably wherein the cross-linking agent in component (B) is or comprises Ca²⁺, preferably wherein the cross-linking agent in component (B) is or comprises Mg²⁺.

Preferably, the or one polymer in component (A) is or comprises chitosan, preferably wherein the cross-linking agent in component (B) is or comprises Cu²⁺, preferably wherein the cross-linking agent in component (B) is or comprises Zn²⁺, preferably wherein the cross-linking agent in component (B) is or comprises Fe³⁺.

Preferably, the or one polymer in component (A) is or comprises hyaluronic acid, preferably wherein the cross-linking agent in component (B) is or comprises Fe³⁺, preferably wherein the cross-linking agent in component (B) is or comprises Al³⁺.

Preferably, the or one polymer in component (A) is or comprises fibrin, preferably wherein the cross-linking agent in component (B) is or comprises Ca²⁺.

Preferably, the or one polymer in component (A) is or comprises gum arabicum, preferably wherein the cross-linking agent in component (B) is or comprises Ca²⁺, preferably wherein the cross-linking agent in component (B) is or comprises Fe³⁺, preferably wherein the cross-linking agent in component (B) is or comprises Al³⁺.

Preferably, the or one polymer in component (A) is or comprises gellan gum, preferably wherein the cross-linking agent in component (B) is or comprises Ca²⁺, preferably wherein the cross-linking agent in component (B) is or comprises Fe³⁺, preferably wherein the cross-linking agent in component (B) is or comprises Al³⁺.

Preferably, the or one polymer in component (A) is or comprises xanthan, preferably wherein the cross-linking agent in component (B) is or comprises Ca²⁺, preferably wherein the cross-linking agent in component (B) is or comprises Mg²⁺.

Preferably, the or one polymer in component (A) is or comprises gelatine, preferably wherein the cross-linking agent in component (B) is or comprises Ca²⁺, preferably wherein the cross-linking agent in component (B) is or comprises Zn²⁺.

Preferably, the or one polymer in component (A) is or comprises pullulan, preferably wherein the cross-linking agent in component (B) is or comprises Fe³⁺, preferably wherein the cross-linking agent in component (B) is or comprises Al³⁺.

Preferably, the or one polymer in component (A) is or comprises fucoidane, preferably wherein the cross-linking agent in component (B) is or comprises Ca²⁺, preferably wherein the cross-linking agent in component (B) is or comprises Mg²⁺, preferably wherein the cross-linking agent in component (B) is or comprises Fe³⁺.

Preferably, the or one polymer in component (A) is or comprises lignin, preferably wherein the cross-linking agent in component (B) is or comprises Fe³⁺, preferably wherein the cross-linking agent in component (B) is or comprises Al³⁺.

Preferably, the or one polymer in component (A) is or comprises agarose, preferably wherein the cross-linking agent in component (B) is or comprises Ca²⁺, preferably wherein the cross-linking agent in component (B) is or comprises Mg²⁺.

Preferably, the or one polymer in component (A) is or comprises dextran, preferably wherein the cross-linking agent in component (B) is or comprises Ca²⁺, preferably wherein the cross-linking agent in component (B) is or comprises Fe³⁺.

Preferably, the or one polymer in component (A) is or comprises amylose, preferably wherein the cross-linking agent in component (B) is or comprises Ca²⁺, preferably wherein the cross-linking agent in component (B) is or comprises Mg²⁺.

Preferably, the or one polymer in component (A) is or comprises chondroitin sulfate, preferably wherein the cross-linking agent in component (B) is or comprises Ca²⁺, preferably wherein the cross-linking agent in component (B) is or comprises Fe³⁺, preferably wherein the cross-linking agent in component (B) is or comprises Al³⁺.

Preferably, the or one polymer in component (A) is or comprises keratan sulfate, preferably wherein the cross-linking agent in component (B) is or comprises Ca²⁺, preferably wherein the cross-linking agent in component (B) is or comprises Mg²⁺.

Preferably, the or one polymer in component (A) is or comprises heparin, preferably wherein the cross-linking agent in component (B) is or comprises Cu²⁺, preferably wherein the cross-linking agent in component (B) is or comprises Fe³⁺, preferably wherein the cross-linking agent in component (B) is or comprises Al³⁺.

Preferably, the or one polymer in component (A) is or comprises lentinan, preferably wherein the cross-linking agent in component (B) is or comprises Ca²⁺, preferably wherein the cross-linking agent in component (B) is or comprises Zn²⁺.

Preferably, the or one polymer in component (A) is or comprises glucomannan, preferably glucomannan from the Konjac plant, preferably wherein the cross-linking agent in component (B) is or comprises Ca²⁺, preferably wherein the cross-linking agent in component (B) is or comprises Mg²⁺.

Preferably, the term "glucomannan", as used herein, refers to or comprises glucomannan from the Konjac plant (*Amorphophallus konjac*)*.*

Preferably, the or one polymer in component (A) is or comprises polygalacturonic acid, preferably wherein the cross-linking agent in component (B) is or comprises Ca²⁺.

Preferably, the or one polymer in component (A) is or comprises sporopollenin, preferably wherein the cross-linking agent in component (B) is or comprises Ca²⁺, preferably wherein the cross-linking agent in component (B) is or comprises Fe³⁺.

Preferably, the term "the cross-linking agent in component (B) is or comprises Ca²⁺", as used herein, describes that Ca2+ is present in the cross-linking agent either as ions, as pharmaceutically acceptable salt(s), or combinations thereof. The same applies accordingly to any other ions.

The term "site of interest", as used herein, preferably refers to the or an area or region of the subject's body at which the medical condition to be treated or prevented is / will be located.

Preferably, the administration of component (A) and component (B) to a site of interest on or in the subject's body can be performed by any possible means considered suitable by a skilled person. Particularly preferably, the administration is performed with a catheter of any type, e.g. an endoscope.

Preferably, the term "completely dissolved" describes that after dissolving the medical drug no remaining particulates or turbidities of the medical drug are visible, at least not visible by eye.

Preferably, the term "partially dissolved" describes that at least 80 wt.-%, preferably at least 85 wt.-%, further preferably at least 87.5 wt.-%, more preferably at least 90 wt.-%, particularly preferably at least 92.5 wt.-%, especially preferably at least 95 wt.-%, even further preferably at least 97.5 wt.-% of the medical drug, based on the total weight of the admixed medical drug, is dissolved.

It is preferred for the gel-forming composition according to the invention that the, one, two, three or more or all medical drug(s) is/are selected from the group consisting of anti-cancer agents, anti-inflammatory agents, analgesics, antibiotics, anti-fungal agents, anti-viral agents, cholesterol-blocking drugs, arachidonic acid inhibitors, immunosuppressants, enzymes, antibodies,

and that the medical condition is selected from the group consisting of cancer, inflammation, pain, bacterial infection, fungal infection, viral infection, hypercholesterolemia, and inflammatory bowel disease.

A skilled person knows that e.g. cancer is treated or prevented with anti-cancer agents. The same applies accordingly to the further medical drugs and conditions mentioned above.

Thus, preferably, the medical drug is or comprises an anti-cancer agent and the medical condition is cancer, preferably kidney or bladder cancer.

Thus, preferably, the medical drug is or comprises an anti-inflammatory agent and the medical condition is inflammation, preferably inflammatory bowel disease.

Thus, preferably, the medical drug is or comprises an analgesic and the medical condition is pain.

Thus, preferably, the medical drug is or comprises an antibiotic and the medical condition is a bacterial infection.

Thus, preferably, the medical drug is or comprises an anti-fungal agent and the medical condition is a fungal infection.

Thus, preferably, the medical drug is or comprises an anti-viral agent and the medical condition is a viral infection.

Thus, preferably, the medical drug is or comprises an cholesterol-blocking drug and the medical condition is hypercholesterolemia.

Thus, preferably, the medical drug is or comprises an arachidonic acid inhibitor and the medical condition is inflammation, preferably inflammatory bowel disease.

Thus, preferably, the medical drug is or comprises an immunosuppressant and the medical condition is inflammation, preferably inflammatory bowel disease.

It is preferred for the gel-forming composition according to the invention that the, one, two, three or more or all medical drug(s) is/are selected from the group consisting of Abraxane, Actinomycin, Alitretinoin, All-trans retinoic acid, Altretamine, Azacitidine, Azathioprine, Belotecan, Bendamustine, Bexarotene, Bleomycin, Bortezomib, Busulfan, Cabazitaxel, Camptothecin, Carboplatin, Carboquone, Carmustine, Capecitabine, Cisplatin, Chlorambucil, Chlormethine, Chlorozotocin, Cladribine, Clofarabine, Cyclophosphamide, Cytarabine, Dacarbazine, Dasatinib, Daunorubicin, Decitabine, Docetaxel, Doxifluridine, Doxorubicin, Epirubicin, Epothilone, Erlotinib, Etoposide, Exatecan, Fludara-bine, Fluorouracil, Fotemustine, Gefitinib, Gemcitabine, Gimatecan, Hydroxyurea, Idarubicin, Ifosfamide, Imatinib, Irinotecan, Ixabepilone, Larotaxel, Lenvatinib, Lomustine, Melpha-lan, Melphalan flufenamide, Mercaptopurine, Methotrexate, Mitobronitol, Mitomycin C, Mitoxantrone, Nelarabine, Nimustine, Nitrosoureas, Oxaliplatin, Paclitaxel, Pemetrexed, Pipobroman, Procarbazine, Ranimustine, Romidepsin, Semustine, Sorafenib, Streptozotocin, Tafluposide, Taxotere, Temoporfin, Temozolomide, Tesetaxel, Teniposide, Thiotepa, Tioguanine, Topotecan, Trabectedin, Treosulfan, Tretinoin, Triaziquone, Triethylenemelamine, Valrubicin, Vemurafenib, Vinblastine, Vincristine, Vindesine, Vinorelbine, Vismodegib, Vorinostat,

preferably mitosan derivatives, particularly preferably mitomycins, further preferably mitomycin A, further preferably mitomycin B, further preferably mitomycin C, further preferably mitomycin D, further preferably mitomycin E, further preferably mitomycin F, further preferably mitomycin G, further preferably mitomycin H, further preferably mitomycin I, further preferably mitomycin J, further preferably mitomycin K,
particularly preferably mitomycin C,
and wherein the medical condition is cancer.

It is preferred for the gel-forming composition according to the invention that the or one cross-linking agent is selected from Ca²⁺, pharmaceutically acceptable salts thereof, and mixtures thereof, particularly wherein the or one cross-linking agent is or comprises CaCl₂.

Optionally, the gel-forming composition according to the invention further comprises one or more dye(s), wherein
the dye(s) is/are a component of component (A) and/or component of component (B),
   and/or
wherein the composition further comprises component (C), which contains one or more dye(s).

Optionally, component (A) and component (B) further comprise one or more dye(s), optionally wherein the dye(s) in component (A) is/are different from the dye(s) in component (B). This has proven to be advantageous, in particular, in that it allows a colour distinction to be made between components (A) and (B). It is also advantageously possible in this way to determine whether components (A) and (B) have already mixed, since mixing the two components typically results in a colour that is different from the colour of the separate components (A) and/or (B).

Preferably, the phrase "wherein the dye(s) in component (A) is different from the dye(s) in component (B)" means that in the case of multiple dyes in one or both of components (A) and (B), the combination of dyes of one component is not the same as the combination of dye(s). Consequently, both components may contain the same dye(s) as long as at least one of the components (A) or (B) also contains another, different dye.

Preferably, the one or more dye is or comprises a stained molecule selected from the group consisting of dextrans, polyehtlyene glycol, starch, gellans, polyelectrolytes such as poly-I-lysine and other poly-amino acids such as poly(ornithine), poly(arginine), poly(histidine); polypeptides and proteins such as gelatin, as well as polysaccharides such as chitosan, alginates such as sodium alginate, poly(glycolic acid) (PGA), poly(lactic acid) (PLA), poly-2-hydroxy butyrate (PHB), polycaprolactone (PCL), poly(lactic-co-glycolic) acid (PLGA), protamine sulfate, spermidine, albumin, carrageenan, furcellarane, pectin, xanthan, hyaluronic acid, sodium carboxymethyl cellulose, heparin, heparane sulfate, cellulose derivatives or sodium carboxymethyl cellulose, chondroitine sulfate, dermatan sulfate and dextran sulfate.

Particularly preferably, such a molecule is stained with at least one of Cibacronblue (F3G-A), C.I. Reactive Blue 235, C.I. Reactive Blue 204, C.I. Reactive Blue 7, C.I. Reactive Red 1, Cibacron Orange G-E, Zibakronbrillantblue FBR-P, Zibakronblue F3G-A, Zibakronbrillantred 3B-A, Zibakronbrown 3GR-A, Zibakronscarlet red 2G, Zibakronscarlet red 4G-P, Zibakronbrillantred B-A, Procionbrillant Orange HGRS, Procion-Blue HBS, Procion-Brillant-red H7BS, Procion-Orange-Brown HGS, Procion-Scarlet red H3GS, Procion-Red H3B, Procion-Red HE2B, Procion-Red P3BN, Procion-Red MX2B, Procion-Blau MX3G, Procion-Yellow MXR, Procion-Yellow H5G, Procion-Red H8BN, Procion-Green H-4G, Procion-Brown H-GR, Procion-Blue MX-G, Procion-Blue HE-RD, Procion-Blue H-B, Procion-Blue MXR, Procion-Yellow HA, Procion-Green HE-4BD, Procion-Red HE7B, Procion-Red MX5B, Procion-Red MY. 8 B, Procion Rubin MXB, Procion Scarlet red MXG, Procion Orange MXG, Procion Yellow MX6G, Procion Brown H2G, Procion Yellow MX8G, Procion Turquoise HA, Procion Turquoise H7G, Procion Brown MX5BR, Procion Blue MX7RX, Procion Blue MX4G.

Preferably, the one or more dye is or comprises dextran blue. Preferably, the one or more dye is or comprises riboflavin.

It is preferred for the gel-forming composition according to the invention that component (A) comprises at least 50 wt.-%, preferably at least 60 wt.-%, particularly preferably at least 70 wt.-%, further preferable at least 75 wt.-%, especially preferably at least 80 wt.-%, more preferably at least 85 wt.-%, even further preferably at least 90 wt.-%, further particularly preferably at least 95 wt.-% of the cross-linkable polymer(s), based on the total weight of component (A).

It is preferred for the gel-forming composition according to the invention that the concentration of the cross-linking agent(s) present in component (B), related to the total volume of component (B), is at least 0.1 mol/L and is preferably in the range of from 0.1 to 3.0 mol/L, particularly preferably in the range of from 0.1 to 1.0 mol/L.

It is preferred for the gel-forming composition according to the invention that component (A) has a neutral pH value, preferably a pH value in the range of from 6.5 to 8.0, particularly preferably a pH in the range of from 7.0 to 7.5.

It is preferred for the gel-forming composition according to the invention that component (B) has a neutral pH value, preferably a pH value in the range of from 6.5 to 8.0, particularly preferably a pH in the range of from 7.0 to 7.5.

It is preferred for the gel-forming composition according to the invention that all components have a neutral pH value, preferably a pH value in the range of from 6.5 to 8.0, particularly preferably a pH in the range of from 7.0 to 7.5.

It is further preferred for the gel-forming composition according to the invention that the composition further comprises magnetisable particles, wherein
the magnetisable particles are a component of component (A) and/or component of component (B),
   and/or wherein
the composition further comprises component (C), which contains magnetisable particles,

preferably wherein the magnetisable particles are selected from particles comprising or consisting of ferromagnetic element(s) such as iron, nickel, and cobalt, as well as alloys such as AlNiCo, SmCo, Nd₂Fe₁₄B, Ni₈₀Fe₂₀, NiFeCo, and/or their oxides such as iron oxide particles, particularly iron oxide particles from Fe₃O₄ and/or γ-Fe₂O₃,
preferably wherein the total amount of the magnetisable particles, related to the total weight of the components (A), (B), or respectively, (C) or (D) containing the magnetisable particles, is at least 0.1 wt.-% and is preferably in the range of from 0.1 to 70 wt.-%, particularly preferably in the range of from 1 to 11 wt.-%.

Preferably, the subject is a human or an animal, preferably a human, preferably an animal.

The present invention further relates to a gel-forming multi-component composition according to the invention for the manufacture of a medicament.

What was said above with regard to the composition and/or to the medicament or, respectively, the medical drug, particularly the embodiments and preferred features, applies accordingly for the composition for the manufacture of a medicament.

Preferably the medicament comprises component (A) and component (B), each as described herein.

Preferably, the medicament comprises the medical drug, as described herein.

Preferably, the medicament is a gel-forming composition, as described herein, wherein the medical drug, as described herein, is partially or completely dissolved in component (A) and/or component (B). The medicament may be used for forming a gel at a site of interest on or in a subject's body, which may continuously release the medical drug.

Preferably, the, one, two, three or more or all medical drug(s) is/are selected from the group consisting of anti-cancer agents, anti-inflammatory agents, analgesics, antibiotics, anti-fungal agents, anti-viral agents, cholesterol-blocking drugs, arachidonic acid inhibitors, immunosuppressants, enzymes, antibodies,

and the medicament is selected from the group consisting of anti-cancer medicaments, anti-inflammation medicaments, analgesics, antibiotics, anti-fungal medicaments, anti-viral medicaments, medicaments against hypercholesterolemia, and medicaments against inflammatory bowel disease.

Preferably, the medicament is used for the same purpose as the medical drug. Thus, if the medical drug is e.g. an anti-cancer agent, the medicament is used against cancer. The same applies accordingly for the further possible types of medical drugs.

Preferably, the medical drug is or comprises an anti-cancer agent and the medicament is an anti-cancer medicament, preferably a medicament against kidney or bladder cancer.

Preferably, the medical drug is or comprises an anti-inflammatory agent and the medicament is an anti-inflammation medicament, preferably a medicament against inflammatory bowel disease.

Preferably, the medical drug is or comprises an analgesic and the medicament is an analgesic.

Preferably, the medical drug is or comprises an antibiotic and the medicament is an antibiotic.

Preferably, the medical drug is or comprises an anti-fungal agent and the medicament is an anti-fungal medicament.

Preferably, the medical drug is or comprises an anti-viral agent and the medicament is an anti-viral medicament.

Preferably, the medical drug is or comprises an cholesterol-blocking drug and the medicament is a medicament against hypercholesterolemia.

Preferably, the medical drug is or comprises an arachidonic acid inhibitor and the medicament is an anti-inflammation medicament, preferably a medicament against inflammatory bowel disease.

Preferably, the medical drug is or comprises an immunosuppressant and the medicament is an anti-inflammation medicament, preferably a medicament against inflammatory bowel disease.

Particularly preferably, the medicament is an anti-cancer agent and the or one medical drug in the composition is selected from the group consisting of Abraxane, Actinomycin, Alitretinoin, All-trans retinoic acid, Altretamine, Azacitidine, Azathioprine, Belotecan, Bendamustine, Bexarotene, Bleomycin, Bortezomib, Busulfan, Cabazitaxel, Camptothecin, Carboplatin, Carboquone, Carmustine, Capecitabine, Cisplatin, Chlorambucil, Chlormethine, Chlorozotocin, Cladribine, Clofarabine, Cyclophosphamide, Cytarabine, Dacarbazine, Dasatinib, Daunorubicin, Decitabine, Docetaxel, Doxifluridine, Doxorubicin, Epirubicin, Epothilone, Erlotinib, Etoposide, Exatecan, Fludara-bine, Fluorouracil, Fotemustine, Gefitinib, Gemcitabine, Gimatecan, Hydroxyurea, Idarubicin, Ifosfamide, Imatinib, Irinotecan, Ixabepilone, Larotaxel, Lenvatinib, Lomustine, Melpha-lan, Melphalan flufenamide, Mercaptopurine, Methotrexate, Mitobronitol, Mitomycin C, Mitoxantrone, Nelarabine, Nimustine, Nitrosoureas, Oxaliplatin, Paclitaxel, Pemetrexed, Pipobroman, Procarbazine, Ranimustine, Romidepsin, Semustine, Sorafenib, Streptozotocin, Tafluposide, Taxotere, Temoporfin, Temozolomide, Tesetaxel, Teniposide, Thiotepa, Tioguanine, Topotecan, Trabectedin, Treosulfan, Tretinoin, Triaziquone, Triethylenemelamine, Valrubicin, Vemurafenib, Vinblastine, Vincristine, Vindesine, Vinorelbine, Vismodegib, Vorinostat,
preferably mitosan derivatives, particularly preferably mitomycins, further preferably mitomycin A, further preferably mitomycin B, further preferably mitomycin C, further preferably mitomycin D, further preferably mitomycin E, further preferably mitomycin F, further preferably mitomycin G, further preferably mitomycin H, further preferably mitomycin I, further preferably mitomycin J, further preferably mitomycin K,
particularly preferably mitomycin C.

The present invention further relates to a method for treating or preventing a medical condition of a subject, wherein the treatment or prevention comprises
i) providing component (A) and component (B) and optionally further component(s), each as defined herein,
ii) providing one or more medical drug(s), as defined herein,
iii) completely or partially dissolving the one or more medical drug(s) in component (A) and/or component (B),
iv) administering the components (A) and (B) and optionally further component(s) as provided in step i) and at least one of components (A) and B) as obtained after step iii) to a site of interest on or in the subject's body,
wherein at least components (A) and (B) are administered separately, such that they mix at the site of interest on or in the subject's body.

What was said above with regard to the composition and/or to the medicament or, respectively, the medical drug and/or to the composition for the manufacture of a medicament, particularly the embodiments and preferred features, applies accordingly for the method for treating or preventing a medical condition.

As described herein, component (A) comprises one or more cross-linkable, preferably cationically cross-linkable polymer(s), wherein the or one polymer is selected from the group consisting of alginates, pectin, carrageenane, gellane, chitosan, hyaluronic acid, fibrin, gum arabicum, gellan gum, xanthan, gelatine, pullulan, fucoidane, lignin, agarose, dextran, amylose, chondroitin sulfate, keratan sulfate, heparin, lentinan, glucomannan, polygalacturonic acid, and sporopollenin.

As described herein, component (B) comprises one or more cross-linking agent(s) for cross-linking the cross-linkable polymer(s) wherein the, one, more or all cross-linking agent(s) of component (B) is/are selected from the group consisting of mono-, bi- or tri-valent cations, preferably from iron, zinc, magnesium, aluminium, potassium and calcium ions, and their pharmaceutically acceptable salts, and mixtures thereof.

As described herein, it is preferred that the, one, two, three or more or all medical drug(s) is/are selected from the group consisting of anti-cancer agents, anti-inflammatory agents, analgesics, antibiotics, anti-fungal agents, anti-viral agents, cholesterol-blocking drugs, arachidonic acid inhibitors, immunosuppressants, enzymes, antibodies.

As described herein, it is preferred that the medical condition is selected from the group consisting of cancer, inflammation, pain, bacterial infection, fungal infection, viral infection, hypercholesterolemia, and inflammatory bowel disease.

A skilled person knows that e.g. cancer is treated or prevented with anti-cancer agents. The same applies accordingly to the further medical drugs and conditions mentioned above.

Thus, preferably, the medical drug is or comprises an anti-cancer agent and the medical condition is cancer, preferably kidney or bladder cancer.

Thus, preferably, the medical drug is or comprises an anti-inflammatory agent and the medical condition is inflammation, preferably inflammatory bowel disease.

Thus, preferably, the medical drug is or comprises an analgesic and the medical condition is pain.

Thus, preferably, the medical drug is or comprises an antibiotic and the medical condition is a bacterial infection.

Thus, preferably, the medical drug is or comprises an anti-fungal agent and the medical condition is a fungal infection.

Thus, preferably, the medical drug is or comprises an anti-viral agent and the medical condition is a viral infection.

Thus, preferably, the medical drug is or comprises an cholesterol-blocking drug and the medical condition is hypercholesterolemia.

Thus, preferably, the medical drug is or comprises an arachidonic acid inhibitor and the medical condition is inflammation, preferably inflammatory bowel disease.

Thus, preferably, the medical drug is or comprises an immunosuppressant and the medical condition is inflammation, preferably inflammatory bowel disease.

It is preferred that the medical condition is cancer and wherein the medical drug is selected from the group consisting of Abraxane, Actinomycin, Alitretinoin, All-trans retinoic acid, Altretamine, Azacitidine, Azathioprine, Belotecan, Bendamustine, Bexarotene, Bleomycin, Bortezomib, Busulfan, Cabazitaxel, Camptothecin, Carboplatin, Carboquone, Carmustine, Capecitabine, Cisplatin, Chlorambucil, Chlormethine, Chlorozotocin, Cladribine, Clofarabine, Cyclophosphamide, Cytarabine, Dacarbazine, Dasatinib, Daunorubicin, Decitabine, Docetaxel, Doxifluridine, Doxorubicin, Epirubicin, Epothilone, Erlotinib, Etoposide, Exatecan, Fludara-bine, Fluorouracil, Fotemustine, Gefitinib, Gemcitabine, Gimatecan, Hydroxyurea, Idarubicin, Ifosfamide, Imatinib, Irinotecan, Ixabepilone, Larotaxel, Lenvatinib, Lomustine, Melpha-lan, Melphalan flufenamide, Mercaptopurine, Methotrexate, Mitobronitol, Mitomycin C, Mitoxantrone, Nelarabine, Nimustine, Nitrosoureas, Oxaliplatin, Paclitaxel, Pemetrexed, Pipobroman, Procarbazine, Ranimustine, Romidepsin, Semustine, Sorafenib, Streptozotocin, Tafluposide, Taxotere, Temoporfin, Temozolomide, Tesetaxel, Teniposide, Thiotepa, Tioguanine, Topotecan, Trabectedin, Treosulfan, Tretinoin, Triaziquone, Triethylenemelamine, Valrubicin, Vemurafenib, Vinblastine, Vincristine, Vindesine, Vinorelbine, Vismodegib, Vorinostat,
preferably mitosan derivatives, particularly preferably mitomycins, further preferably mitomycin A, further preferably mitomycin B, further preferably mitomycin C, further preferably mitomycin D, further preferably mitomycin E, further preferably mitomycin F, further preferably mitomycin G, further preferably mitomycin H, further preferably mitomycin I, further preferably mitomycin J, further preferably mitomycin K,
particularly preferably mitomycin C.

Preferably, the term "completely dissolving" describes that after dissolving the medical drug no remaining particulates or turbidities of the medical drug are visible, at least not visible by eye.

Preferably, the term "partially dissolving" describes that at least 80 wt.-%, preferably at least 85 wt.-%, further preferably at least 87.5 wt.-%, more preferably at least 90 wt.-%, particularly preferably at least 92.5 wt.-%, especially preferably at least 95 wt.-%, even further preferably at least 97.5 wt.-% of the medical drug, based on the total weight of the admixed medical drug, is dissolved.

Preferably, the administration of component (A) and component (B) to a site of interest on or in the subject's body can be performed by any possible means considered suitable by a skilled person. Particularly preferably, the administration is performed with a catheter of any type.

The present invention further relates to the use of component (A), as defined herein, or component (B), as defined herein, for dissolving at least one medical drug as defined herein.

What was said above with regard to the composition and/or to the medicament or, respectively, the medical drug and/or to the composition for the manufacture of a medicament, and/or to the method of treating or preventing, particularly the embodiments and preferred features, applies accordingly for the use according to the invention.

As described herein, component (A) comprises one or more cross-linkable, preferably cationically cross-linkable polymer(s), wherein the or one polymer is selected from the group consisting of alginates, pectin, carrageenane, gellane, chitosan, hyaluronic acid, fibrin, gum arabicum, gellan gum, xanthan, gelatine, pullulan, fucoidane, lignin, agarose, dextran, amylose, chondroitin sulfate, keratan sulfate, heparin, lentinan, glucomannan, polygalacturonic acid, and sporopollenin.

As described herein, component (B) comprises one or more cross-linking agent(s) for cross-linking the cross-linkable polymer(s) wherein the, one, more or all cross-linking agent(s) of component (B) is/are selected from the group consisting of mono-, bi- or tri-valent cations, preferably from iron, zinc, magnesium, aluminium, potassium and calcium ions, and their pharmaceutically acceptable salts, and mixtures thereof.

For example, it is preferred that the or one polymer in component (A) is or comprises an alginate, preferably sodium alginate, preferably wherein the cross-linking agent in component (B) is or comprises Ca²⁺. Further preferred polymer(s) and/or cross-linking agents are described herein, e.g. above.

As described herein, it is preferred that the, one, two, three or more or all medical drug(s) is/are selected from the group consisting of anti-cancer agents, anti-inflammatory agents, analgesics, antibiotics, anti-fungal agents, anti-viral agents, cholesterol-blocking drugs, arachidonic acid inhibitors, immunosuppressants, enzymes, antibodies.

It is preferred that the medical drug is selected from the group consisting of Abraxane, Actinomycin, Alitretinoin, All-trans retinoic acid, Altretamine, Azacitidine, Azathioprine, Belotecan, Bendamustine, Bexarotene, Bleomycin, Bortezomib, Busulfan, Cabazitaxel, Camptothecin, Carboplatin, Carboquone, Carmustine, Capecitabine, Cisplatin, Chlorambucil, Chlormethine, Chlorozotocin, Cladribine, Clofarabine, Cyclophosphamide, Cytarabine, Dacarbazine, Dasatinib, Daunorubicin, Decitabine, Docetaxel, Doxifluridine, Doxorubicin, Epirubicin, Epothilone, Erlotinib, Etoposide, Exatecan, Fludara-bine, Fluorouracil, Fotemustine, Gefitinib, Gemcitabine, Gimatecan, Hydroxyurea, Idarubicin, Ifosfamide, Imatinib, Irinotecan, Ixabepilone, Larotaxel, Lenvatinib, Lomustine, Melpha-lan, Melphalan flufenamide, Mercaptopurine, Methotrexate, Mitobronitol, Mitomycin C, Mitoxantrone, Nelarabine, Nimustine, Nitrosoureas, Oxaliplatin, Paclitaxel, Pemetrexed, Pipobroman, Procarbazine, Ranimustine, Romidepsin, Semustine, Sorafenib, Streptozotocin, Tafluposide, Taxotere, Temoporfin, Temozolomide, Tesetaxel, Teniposide, Thiotepa, Tioguanine, Topotecan, Trabectedin, Treosulfan, Tretinoin, Triaziquone, Triethylenemelamine, Valrubicin, Vemurafenib, Vinblastine, Vincristine, Vindesine, Vinorelbine, Vismodegib, Vorinostat,
preferably mitosan derivatives, particularly preferably mitomycins, further preferably mitomycin A, further preferably mitomycin B, further preferably mitomycin C, further preferably mitomycin D, further preferably mitomycin E, further preferably mitomycin F, further preferably mitomycin G, further preferably mitomycin H, further preferably mitomycin I, further preferably mitomycin J, further preferably mitomycin K,
particularly preferably mitomycin C.

Preferably, in the use according to the invention, components (A) and (B) are not in contact with each other when the at least one medical drug is dissolved.

Preferably, the term "dissolving", as used for the use according to the invention, refers to completely or partially dissolving the at least one medical drug.

Preferably, the term "completely dissolved" describes that after dissolving the medical drug no remaining particulates or turbidities of the medical drug are visible, at least not visible by eye.

Preferably, the term "partially dissolved" describes that at least 80 wt.-%, preferably at least 85 wt.-%, further preferably at least 87.5 wt.-%, more preferably at least 90 wt.-%, particularly preferably at least 92.5 wt.-%, especially preferably at least 95 wt.-%, even further preferably at least 97.5 wt.-% of the medical drug, based on the total weight of the admixed medical drug, is dissolved.

The present invention further relates to a method for dissolving at least one medical drug as defined herein, in component (A), as defined herein, or component (B), as defined herein, the method comprising
- providing one or more medical drug(s), as defined herein,
- providing component (A) or component (B), each as defined herein,
- dissolving the provided medical drug(s) in provided component (A) or (B).

What was said above with regard to the composition and/or to the medicament or, respectively, the medical drug and/or to the composition for the manufacture of a medicament, and/or to the method of treating or preventing and/or the use according to the invention, particularly the embodiments and preferred features, applies accordingly for the method for dissolving according to the invention.

As described herein, component (A) comprises one or more cross-linkable, preferably cationically cross-linkable polymer(s), wherein the or one polymer is selected from the group consisting of alginates, pectin, carrageenane, gellane, chitosan, hyaluronic acid, fibrin, gum arabicum, gellan gum, xanthan, gelatine, pullulan, fucoidane, lignin, agarose, dextran, amylose, chondroitin sulfate, keratan sulfate, heparin, lentinan, glucomannan, polygalacturonic acid, and sporopollenin.

As described herein, component (B) comprises one or more cross-linking agent(s) for cross-linking the cross-linkable polymer(s) wherein the, one, more or all cross-linking agent(s) of component (B) is/are selected from the group consisting of mono-, bi- or tri-valent cations, preferably from iron, zinc, magnesium, aluminium, potassium and calcium ions, and their pharmaceutically acceptable salts, and mixtures thereof.

Preferred polymer(s) and/or cross-linking agents are described herein, e.g. above.

For example, it is preferred that the or one polymer in component (A) is or comprises an alginate, preferably sodium alginate.

For example, it is preferred that the cross-linking agent in component (B) is or comprises Ca²⁺.

As described herein, it is preferred that the, one, two, three or more or all medical drug(s) is/are selected from the group consisting of anti-cancer agents, anti-inflammatory agents, analgesics, antibiotics, anti-fungal agents, anti-viral agents, cholesterol-blocking drugs, arachidonic acid inhibitors, immunosuppressants, enzymes, antibodies.

It is preferred that the medical drug is selected from the group consisting of Abraxane, Actinomycin, Alitretinoin, All-trans retinoic acid, Altretamine, Azacitidine, Azathioprine, Belotecan, Bendamustine, Bexarotene, Bleomycin, Bortezomib, Busulfan, Cabazitaxel, Camptothecin, Carboplatin, Carboquone, Carmustine, Capecitabine, Cisplatin, Chlorambucil, Chlormethine, Chlorozotocin, Cladribine, Clofarabine, Cyclophosphamide, Cytarabine, Dacarbazine, Dasatinib, Daunorubicin, Decitabine, Docetaxel, Doxifluridine, Doxorubicin, Epirubicin, Epothilone, Erlotinib, Etoposide, Exatecan, Fludara-bine, Fluorouracil, Fotemustine, Gefitinib, Gemcitabine, Gimatecan, Hydroxyurea, Idarubicin, Ifosfamide, Imatinib, Irinotecan, Ixabepilone, Larotaxel, Lenvatinib, Lomustine, Melpha-lan, Melphalan flufenamide, Mercaptopurine, Methotrexate, Mitobronitol, Mitomycin C, Mitoxantrone, Nelarabine, Nimustine, Nitrosoureas, Oxaliplatin, Paclitaxel, Pemetrexed, Pipobroman, Procarbazine, Ranimustine, Romidepsin, Semustine, Sorafenib, Streptozotocin, Tafluposide, Taxotere, Temoporfin, Temozolomide, Tesetaxel, Teniposide, Thiotepa, Tioguanine, Topotecan, Trabectedin, Treosulfan, Tretinoin, Triaziquone, Triethylenemelamine, Valrubicin, Vemurafenib, Vinblastine, Vincristine, Vindesine, Vinorelbine, Vismodegib, Vorinostat,
preferably mitosan derivatives, particularly preferably mitomycins, further preferably mitomycin A, further preferably mitomycin B, further preferably mitomycin C, further preferably mitomycin D, further preferably mitomycin E, further preferably mitomycin F, further preferably mitomycin G, further preferably mitomycin H, further preferably mitomycin I, further preferably mitomycin J, further preferably mitomycin K,
particularly preferably mitomycin C.

Preferably, in the method for dissolving according to the invention, components (A) and (B) may both be provided, however in this case, components (A) and (B) are not in contact with each other when the at least one medical drug is dissolved.

Preferably, the term "dissolving", as used for the method for dissolving according to the invention, refers to completely or partially dissolving the at least one medical drug.

Preferably, the term "completely dissolved" describes that after dissolving the medical drug no remaining particulates or turbidities of the medical drug are visible, at least not visible by eye.

Preferably, the term "partially dissolved" describes that at least 80 wt.-%, preferably at least 85 wt.-%, further preferably at least 87.5 wt.-%, more preferably at least 90 wt.-%, particularly preferably at least 92.5 wt.-%, especially preferably at least 95 wt.-%, even further preferably at least 97.5 wt.-% of the medical drug, based on the total weight of the admixed medical drug, is dissolved.

Further aspects and advantages of the present invention result from the subsequent description of preferred examples.

### Examples

### Example 1: Dissolving the medical drug

The hardly soluble Mitomycin C was used as medical drug.

In a first approach, 10 mg Mitomycin C were provided and successfully dissolved in 10 ml physiological saline solution (0.9 wt.-% NaCl). The provided Mitomycin C was dissolved within a few minutes.

In a second approach, 10 mg Mitomycin C and a composition containing 4 g sodium alginate per liter water were provided.

5 mL of the composition were used to dissolve Mitomycin C. The Mitomycin C was dissolved within a few minutes, no difference to the dissolution of the first approach was detected.

Thus, it was surprisingly found that the hardly soluble Mitomycin C could be dissolved in composition (A) according to the invention.

### Example 2: Gel formation with the medical drug

Mitomycin C was used as medical drug.

As exemplary component (A), 4 g sodium alginate were dissolved in 1 liter water.

As exemplary component (B), 10 g calcium chloride dihydrate were dissolved in 1 liter water.

In a first approach, 2 mL of the exemplary component (A) are contacted with 10 ml of the exemplary component (B). Within a few seconds, a gel was formed. The gel could be collected and be lifted with a forceps, thus a stable gel was formed.

In a second approach, 10 mg Mitomycin C were dissolved in 5 ml of the exemplary component (A), as described in Example 1.

2 mL of the exemplary component (A) including the dissolved Mitomycin C are contacted with 10 ml of the exemplary component (B). Within a few seconds, a gel was formed. The gel could be collected and be lifted with a forceps, thus a stable gel was formed. No difference to the gel and gel formation of the first approach was detected.

Thus, including a medical drug does not negatively affect the gel formation or the formed gel.

### Example 3: Degradation of the formed gel

### Example 3.1 First approach, without medical drug

In a first approach, 60 ml of synthetic urine according to DIN EN 1616 / DIN EN ISO 20696 were provided in a first beaker.

In a second beaker, 2 ml of the exemplary component (A) of Example 2 were contacted with 10 ml of the exemplary component (B) of Example 2. As also described in Example 2, a gel was formed within seconds. The gel was collected with a forceps and placed in the synthetic urine in the first beaker.

For 60 min, the consistency of the gel was examined each 10 min with a forceps and evaluated as shown below.

The first approach was performed in duplicates.

After 10 min, the gels could be grabbed and lifted out of the synthetic urine.

After 20 min and after 30 min, the gels could be grabbed and lifted out of the synthetic urine, but less ideal than after 10 min (see above).

After 40 min, the gels could be grabbed but hardly lifted out of the synthetic urine.

After 50 min, the gels could be grabbed but hardly lifted out of the synthetic urine (less ideal than after 40 min, see above).

After 60 min, it was almost impossible to grab and lift the gels out of the synthetic urine.

### Example 3.2 Second approach, with medical drug in the gel

In a second approach, 60 ml of synthetic urine according to DIN EN 1616 / DIN EN ISO 20696 were provided in a first beaker. 10 mg Mitomycin C were dissolved in 5 ml of the exemplary component (A), as described in Example 2.

In a second beaker, 2 mL of the exemplary component (A) including the dissolved Mitomycin C are contacted with 10 ml of the exemplary component (B) of Example 2. As also described in Example 2, a gel was formed within seconds. The gel was collected with a forceps and placed in the synthetic urine in the first beaker.

For 60 min, the consistency of the gel was examined each 10 min with a forceps and evaluated as shown below.

The second approach was performed in duplicates.

After 10 min and after 20 min, the gels could be grabbed and lifted out of the synthetic urine, however, after 20 min, one of the two gels could be hardly lifted out of the synthetic urine.

After 30 min, after 40 min and after 50 min, the gels could be grabbed but hardly lifted out of the synthetic urine.

After 60 min, it was almost impossible to grab and lift the gels out of the synthetic urine.

The results of Example 3.2 and Example 3.1 are very similar. Thus, including a medical drug does not affect the degradation characteristics of the formed gel.

Further, starting after a few minutes, the synthetic urine obtained a slightly blueish, purple colour, which was more intense with each further time point. Such a discoloration was not observed in Example 3.1 It was thus concluded that Mitomycin C was released.

### Example 3.3 Third approach, with medical drug in the synthetic urine

The same approach as described in Example 3.1 was performed. However, 10 mg Mitomycin were dissolved in 10 ml physiological saline solution (0.9 wt.-% NaCl) and added to the synthetic urine.

The third approach was performed in duplicates.

No difference to the consistency of the gel was observed compared to Example 3.1.

Thus, adding the medical drug to the surrounding medium does not affect the gel consistency and its degradation.

## Claims

1. Gel-forming multi-component composition, preferably two-component composition, for forming a gel,
the composition comprising or consisting of
a component (A), comprising one or more cross-linkable, preferably cationically cross-linkable polymer(s),
wherein the or one polymer is selected from the group consisting of alginates, pectin, carrageenane, gellane, chitosan, hyaluronic acid, fibrin, gum arabicum, gellan gum, xanthan, gelatine, pullulan, fuocidane, lignin, agarose, dextran, amylose, chondroitin sulfate, keratan sulfate, heparin, lentinan, glucomannan, polygalaturonic acid, and sporopollenin,
and
a component (B), comprising one or more cross-linking agent(s) for cross-linking the cross-linkable polymer(s)
preferably wherein the, one, more or all cross-linking agent(s) of component (B) is/are selected from the group consisting of mono-, bi- or tri-valent cations, preferably from iron, zinc, magnesium, aluminium, potassium and calcium ions, and their pharmaceutically acceptable salts, and mixtures thereof,
for use in a method for treating or preventing a medical condition of a subject,
wherein the treatment or prevention comprises administering component (A) and component (B) to a site of interest on or in the subject's body,
wherein before the administration at least one medical drug is partially or completely dissolved in component (A) and/or component (B).

2. Gel-forming multi-component composition for use according to claim 1,
wherein the, one, two, three or more or all medical drug(s) is/are selected from the group consisting of anti-cancer agents, anti-inflammatory agents, analgesics, antibiotics, anti-fungal agents, anti-viral agents, cholesterol-blocking drugs, arachidonic acid inhibitors, immunosuppressants, enzymes, antibodies,
and wherein the medical condition is selected from the group consisting of cancer, inflammation, pain, bacterial infection, fungal infection, viral infection, hypercholesterolemia, and inflammatory bowel disease.

3. Gel-forming multi-component composition for use according to any of the preceding claims, wherein the, one, two, three or more or all medical drug(s) is/are selected from the group consisting of Abraxane, Actinomycin, Alitretinoin, All-trans retinoic acid, Altretamine, Azacitidine, Azathioprine, Belotecan, Bendamustine, Bexarotene, Bleomycin, Bortezomib, Busulfan, Cabazitaxel, Camptothecin, Carboplatin, Carboquone, Carmustine, Capecitabine, Cisplatin, Chlorambucil, Chlormethine, Chlorozotocin, Cladribine, Clofarabine, Cyclophosphamide, Cytarabine, Dacarbazine, Dasatinib, Daunorubicin, Decitabine, Docetaxel, Doxifluridine, Doxorubicin, Epirubicin, Epothilone, Erlotinib, Etoposide, Exatecan, Fludara-bine, Fluorouracil, Fotemustine, Gefitinib, Gemcitabine, Gimatecan, Hydroxyurea, Idarubicin, Ifosfamide, Imatinib, Irinotecan, Ixabepilone, Larotaxel, Lenvatinib, Lomustine, Melpha-lan, Melphalan flufenamide, Mercaptopurine, Methotrexate, Mitobronitol, Mitomycin C, Mitoxantrone, Nelarabine, Nimustine, Nitrosoureas, Oxaliplatin, Paclitaxel, Pemetrexed, Pipobroman, Procarbazine, Ranimustine, Romidepsin, Semustine, Sorafenib, Streptozotocin, Tafluposide, Taxotere, Temoporfin, Temozolomide, Tesetaxel, Teniposide, Thiotepa, Tioguanine, Topotecan, Trabectedin, Treosulfan, Tretinoin, Triaziquone, Triethylenemelamine, Valrubicin, Vemurafenib, Vinblastine, Vincristine, Vindesine, Vinorelbine, Vismodegib, Vorinostat,
preferably mitosan derivatives, particularly preferably mitomycins, further preferably mitomycin C,
and wherein the medical condition is cancer.

4. Gel-forming multi-component composition for use according to any of the preceding claims, wherein the or one cross-linking agent is selected from Ca²⁺, pharmaceutically acceptable salts thereof, and mixtures thereof, particularly wherein the or one cross-linking agent is CaCl₂.

5. Gel-forming multi-component composition for use according to any of the preceding claims, wherein the composition further comprises one or more dye(s), wherein
the dye(s) is/are a component of component (A) and/or component of component (B),
and/or
wherein the composition further comprises component (C), which contains one or more dye(s).

6. Gel-forming multi-component composition for use according to any of the preceding claims, wherein the concentration of the cross-linking agent(s) present in component (B), related to the total volume of component (B), is at least 0.1 mol/L and is preferably in the range of from 0.1 to 3.0 mol/L, particularly preferably in the range of from 0.1 to 1.0 mol/L.

7. Gel-forming multi-component composition for use according to any of the preceding claims, wherein component (B) has a neutral pH value, preferably a pH value in the range of from 6.5 to 8.0, particularly preferably a pH in the range of from 7.0 to 7.5.

8. Gel-forming multi-component composition for use according to any of the preceding claims, wherein the composition further comprises magnetisable particles, wherein
the magnetisable particles are a component of component (A) and/or component of component (B),
and/or wherein
the composition further comprises component (C), which contains magnetisable particles,
preferably wherein the magnetisable particles are selected from particles comprising or consisting of ferromagnetic element(s) such as iron, nickel, and cobalt, as well as alloys such as AlNiCo, SmCo, Nd₂Fe₁₄B, Ni₈₀Fe₂₀, NiFeCo, and/or their oxides such as iron oxide particles, particularly iron oxide particles from Fe₃O₄ and/or γ-Fe₂O₃, preferably wherein the total amount of the magnetisable particles, related to the total weight of the components (A), (B), or respectively, (C) containing the magnetisable particles, is at least 0.1 wt.-% and is preferably in the range of from 0.1 to 70 wt.-%, particularly preferably in the range of from 1 to 11 wt.-%.

9. Gel-forming multi-component composition as defined in one of the preceding claims for the manufacture of a medicament.

10. Gel-forming multi-component composition according to claim 9 for the manufacture of a medicament, wherein the or one or more or all medicament(s) is/are selected from the group consisting of anti-cancer medicaments, anti-inflammation medicaments, analgesics, antibiotics, anti-fungal medicaments, anti-viral medicaments, medicaments against hypercholesterolemia, and medicaments against inflammatory bowel disease.

11. Gel-forming multi-component composition according to claim 9 or 10 for the manufacture of a medicament, wherein the medicament is an anti-cancer agent and the or one medical drug in the composition is selected from the group consisting of Abraxane, Actinomycin, Alitretinoin, All-trans retinoic acid, Altretamine, Azacitidine, Azathioprine, Belotecan, Bendamustine, Bexarotene, Bleomycin, Bortezomib, Busulfan, Cabazitaxel, Camptothecin, Carboplatin, Carboquone, Carmustine, Capecitabine, Cisplatin, Chlorambucil, Chlormethine, Chlorozotocin, Cladribine, Clofarabine, Cyclophosphamide, Cytarabine, Dacarbazine, Dasatinib, Daunorubicin, Decitabine, Docetaxel, Doxifluridine, Doxorubicin, Epirubicin, Epothilone, Erlotinib, Etoposide, Exatecan, Fludara-bine, Fluorouracil, Fotemustine, Gefitinib, Gemcitabine, Gimatecan, Hydroxyurea, Idarubicin, Ifosfamide, Imatinib, Irinotecan, Ixabepilone, Larotaxel, Lenvatinib, Lomustine, Melpha-lan, Melphalan flufenamide, Mercaptopurine, Methotrexate, Mitobronitol, Mitomycin C, Mitoxantrone, Nelarabine, Nimustine, Nitrosoureas, Oxaliplatin, Paclitaxel, Pemetrexed, Pipobroman, Procarbazine, Ranimustine, Romidepsin, Semustine, Sorafenib, Streptozotocin, Tafluposide, Taxotere, Temoporfin, Temozolomide, Tesetaxel, Teniposide, Thiotepa, Tioguanine, Topotecan, Trabectedin, Treosulfan, Tretinoin, Triaziquone, Triethylenemelamine, Valrubicin, Vemurafenib, Vinblastine, Vincristine, Vindesine, Vinorelbine, Vismodegib, Vorinostat,
preferably mitosan derivatives, particularly preferably mitomycins, further preferably mitomycin C.

12. Method for treating or preventing a medical condition of a subject, wherein the treatment or prevention comprises
i) providing component (A) and component (B) and optionally further component(s), each as defined in the previous claims,
ii) providing one or more medical drug(s), as defined in the previous claims,
iii) completely or partially dissolving the one or more medical drug(s) in component (A) and/or component (B),
iv) administering the components (A) and (B) and optionally further component(s), as provided in step i) and at least one of components (A) and B) as obtained after step iii), to a site of interest on or in the subject's body,
wherein at least components (A) and (B) are administered separately, such that they mix at the site of interest on or in the subject's body.

13. Method for treating or preventing a medical condition according to claim 12, wherein the medical condition is selected from the group consisting of cancer, inflammation, pain, bacterial infection, fungal infection, viral infection, hypercholesterolemia, and inflammatory bowel disease,
and wherein the, one, two, three or more or all medical drug(s) is/are selected from the group consisting of anti-cancer agents, anti-inflammatory agents, analgesics, antibiotics, anti-fungal agents, anti-viral agents, cholesterol-blocking drugs, arachidonic acid inhibitors, immunosuppressants, enzymes, antibodies.

14. Method for treating or preventing a medical condition according to claim 12 or 13, wherein the medical condition is cancer and wherein the, one, two, three or more or all medical drug(s) is/are selected from the group consisting of Abraxane, Actinomycin, Alitretinoin, All-trans retinoic acid, Altretamine, Azacitidine, Azathioprine, Belotecan, Bendamustine, Bexarotene, Bleomycin, Bortezomib, Busulfan, Cabazitaxel, Camptothecin, Carboplatin, Carboquone, Carmustine, Capecitabine, Cisplatin, Chlorambucil, Chlormethine, Chlorozotocin, Cladribine, Clofarabine, Cyclophosphamide, Cytarabine, Dacarbazine, Dasatinib, Daunorubicin, Decitabine, Docetaxel, Doxifluridine, Doxorubicin, Epirubicin, Epothilone, Erlotinib, Etoposide, Exatecan, Fludara-bine, Fluorouracil, Fotemustine, Gefitinib, Gemcitabine, Gimatecan, Hydroxyurea, Idarubicin, Ifosfamide, Imatinib, Irinotecan, Ixabepilone, Larotaxel, Lenvatinib, Lomustine, Melpha-lan, Melphalan flufenamide, Mercaptopurine, Methotrexate, Mitobronitol, Mitomycin C, Mitoxantrone, Nelarabine, Nimustine, Nitrosoureas, Oxaliplatin, Paclitaxel, Pemetrexed, Pipobroman, Procarbazine, Ranimustine, Romidepsin, Semustine, Sorafenib, Streptozotocin, Tafluposide, Taxotere, Temoporfin, Temozolomide, Tesetaxel, Teniposide, Thiotepa, Tioguanine, Topotecan, Trabectedin, Treosulfan, Tretinoin, Triaziquone, Triethylenemelamine, Valrubicin, Vemurafenib, Vinblastine, Vincristine, Vindesine, Vinorelbine, Vismodegib, Vorinostat,
preferably mitosan derivatives, particularly preferably mitomycins, further preferably mitomycin C.

15. Use of component (A), as defined in any of the preceding claims, or component (B), as defined in any of the preceding claims, for dissolving at least one medical drug as defined in any of the preceding claims.
